# EUROPÄISCHE PATENTANMELDUNG

(11) **EP 3 613 388 A1**
(43) Veröffentlichungstag der Anmeldung: **26.02.2020**
(21) Anmeldenummer: 18190806.2
(22) Anmeldetag: 24.08.2018
(51) Int. Cl.: A61F 2/91, B23K 26/08, B23K 26/364, B23K 26/0622, B23K 101/06

(54) **VERFAHREN UND EINRICHTUNG ZUM LASERSCHNEIDEN, INSBESONDERE ZUM LASERSCHNEIDEN VON STENTS**

(71) Anmelder: BIOTRONIK AG, 8180 Bülach (CH)
(72) Erfinder: REMUND, Stefan, 3005 Bern (CH); KRAMER, Thorsten, 4500 Solothurn (CH); NEUENSCHWANDER, Beat, 3400 Burgdorf (CH); JÄGGI, Beat, 3427 Utzenstorf (CH); HOLTZ, Ronald, 3422 Kirchberg (CH)
(74) Vertreter: Galander, Marcus

(57) **Zusammenfassung**

Die Erfindung betrifft ein Verfahren zum Laserstrahlbearbeiten eines entlang einer Achse (A) erstreckten Werkstücks (15) mittels einer Einrichtung zur Laserstrahlbearbeitung (1), wobei die Einrichtung zur Laserstrahlbearbeitung (1) eine Lasereinrichtung (2) aufweist, die dazu konfiguriert ist, einen Laserstrahl (5) von einzelnen aufeinanderfolgenden Laserlichtpulsen (5a, 5b, 5c) zu erzeugen, eine Strahlablenkeinheit (9), die dazu konfiguriert ist, den Laserstrahl (5) auf eine Oberfläche (15a) des Werkstücks (15) abzulenken und auf der Oberfläche (15a) entlang der Achse (A) zu bewegen, und einen Antrieb (16) zum kontinuierlichen Rotieren des Werkstücks (15) um die Achse (A) in einer Rotationsrichtung (U) mit einer Rotationsgeschwindigkeit, wobei zum Laserstrahlbearbeiten des Werkstücks (15) das Werkstück (15) mittels des Antriebs (16) in eine kontinuierliche Rotation in der Rotationsrichtung (U) um die Rotationsachse (A) versetzt wird und der Laserstrahl (5) mittels der Strahlablenkeinheit (9) auf die Oberfläche (15a) des Werkstücks (15) abgelenkt und auf der Oberfläche (15a) entlang der Achse (A) bewegt wird, wobei Material des Werkstücks (15) mittels Laserlichtpulsen (15a, 15c) abgetragen wird, und wobei die Lasereinrichtung (2) mit der Strahlablenkeinheit (9) und dem Antrieb (16) so synchronisiert ist, dass der jeweilige Laserlichtpuls (15a, 15c) die Oberfläche (15a) in einem gewünschten Auftreffpunkt (15b) der Oberfläche (15a) des Werkstücks (15) trifft.

## Beschreibung

Die vorliegende Erfindung betrifft ein Verfahren zum Laserstrahlschneiden und -bohren, insbesondere zum Laserstrahlschneiden von Stents, sowie eine Einrichtung zum Laserstrahlschneiden, insbesondere zur Durchführung des erfindungsgemäßen Verfahrens.

Fast alle heute verfügbaren Einrichtungen zum Stentschneiden basieren auf einem thermischen Laserstrahl-Schneidprozess. Gepulste oder kontinuierliche Laserstrahlung eines Faserlasers wird dabei durch einen Bearbeitungskopf, eine Festoptik mit Schneiddüse, die die Laserstrahlung fokussiert, auf das Werkstück, typischerweise ein Metall- oder Kunststoffröhrchen, geführt. Dabei kann der Bearbeitungskopf in z-Richtung verfahren werden, um die Fokuslage dem Prozess anzupassen. Das zu bearbeitende Stent-Rohrmaterial kann dabei in der Regel um seine Längsachse rotiert sowie in der Längsrichtung linear verfahren werden.

Das Stent-Rohrmaterial wird dabei üblicherweise in einer Spannzange gehalten und in kurzen Teilstücken bearbeitet. Dabei kann das Stent-Rohrmaterial mit einer Vorrichtung fixiert werden, wenn die Spannzange gelöst wird und das Stent-Rohrmaterial um einen definierten Weg vorgeschoben wird. Hiernach wird die Spannzange wieder geschlossen und das nächste Teilstück des Stent-Rohrmaterials wird bearbeitet.

Aus dem Stent-Rohrmaterial werden insbesondere einzelne geschlossene Flächen ausgeschnitten, deren Außenkonturen mit den beiden Achsen abgefahren werden.
Die Prozessgeschwindigkeit ist im Stand der Technik nicht durch die verfügbare Laserleistung oder Maximalgeschwindigkeit einer der beiden Achsen begrenzt, sondern durch ihre Dynamik, insbesondere die Beschleunigung der Achsen. Zusätzlich müssen bei jeder Fläche, die ausgeschnitten werden soll, ein Einstich und eine sogenannte Anfahne hinzugefügt werden. Somit hängt die Bearbeitungszeit auch von der Stentgeometrie, also von der Anzahl der Flächen ab.

Zum Schneiden von Stents wird in jüngeren Anwendungen, wie z. B. in der EP 3 045 257 beschrieben, auch ultrakurz-gepulste Laserstrahlung verwendet, da diese eine bessere Bearbeitungsqualität und weniger Aufwand durch Nacharbeiten verspricht. Allerdings wird bei derartigen Anwendungen in der Regel die Maschinentechnik nicht adaptiert, so dass zwar eine leicht verbesserte Schnittqualität erreicht wird, aber die Bearbeitungsdauer nicht deutlich verringert werden kann. Da Ultrakurzpulslaser mit signifikant höheren Kosten in der Anschaffung verbunden sind als konventionelle Faserlaser, fällt jede Wirtschaftlichkeitsrechnung negativ aus, da die Bearbeitungsdauer mit der derzeitigen Maschinentechnik nicht deutlich reduziert werden kann.

Darüber hinaus bleibt der so durchgeführte Schneidprozess primär thermisch dominiert, da auch für ultrakurze Pulse durch die niedrigen Vorschubgeschwindigkeiten, die vornehmlich durch die Beschleunigung in den Kurven bestimmt sind, und durch die hohen Repetitionsraten viel Wärme im Werkstück deponiert wird. Daher kann das Potential der ultrakurzen Pulse nicht vollständig ausgeschöpft werden.

Hiervon ausgehend liegt der vorliegenden Erfindung die Aufgabe zugrunde, ein Verfahren und eine Einrichtung zum Laserstrahlbearbeiten bereitzustellen, die ein effizientes und präzises Bearbeiten eines Werkstücks, insbesondere zur Herstellung eines Stents oder einer Gefäßstütze ermöglichen.

Diese Aufgabe wird durch ein Verfahren mit den Merkmalen des Anspruchs 1 sowie durch eine Einrichtung mit den Merkmalen des Anspruchs 15 gelöst.

Ausführungsformen dieser Erfindungsaspekte sind in den entsprechenden Unteransprüchen angegeben und/oder werden nachfolgend beschrieben.

Gemäß Anspruch 1 wird ein Verfahren zum Laserstrahlbearbeiten (insbesondere Laserstrahlschneiden) eines entlang einer Achse erstreckten z. B. rohrförmigen Werkstücks mit einer zu bearbeitenden Oberfläche mittels einer Einrichtung zur Laserstrahlbearbeitung vorgeschlagen, wobei die Einrichtung zur Laserstrahlbearbeitung eine Lasereinrichtung aufweist, die dazu konfiguriert ist, einen Laserstrahl in Form von einzelnen aufeinanderfolgenden Laserlichtpulsen zum Laserstrahlbearbeiten des Werkstücks zu erzeugen, eine Strahlablenkeinheit, z. B. ein Galvanometerscanner, der dazu konfiguriert ist, den Laserstrahl auf die Oberfläche das Werkstücks abzulenken und auf der Oberfläche entlang der Achse zu bewegen, und einen Antrieb zum kontinuierlichen Rotieren des Werkstücks um die Achse in einer Rotationsrichtung mit einer (z. B. konstanten) Rotationsgeschwindigkeit, wobei zum Laserstrahlbearbeiten des Werkstücks das Werkstück mittels des Antriebs in eine kontinuierliche Rotation in der Rotationsrichtung um die Rotationsachse versetzt wird und der Laserstrahl mittels der Strahlablenkeinheit auf die Oberfläche des Werkstücks abgelenkt und auf der Oberfläche entlang der Achse bewegt wird, insbesondere hin- und herbewegt wird, wobei Material des Werkstücks mittels des Laserstrahls bzw. mittels der Laserlichtpulse abgetragen wird, und wobei die Lasereinrichtung mit der Strahlablenkeinheit und dem Antrieb so synchronisiert ist, dass der jeweilige Laserlichtpuls die Oberfläche in einem gewünschten (d.h. vordefinierten) Auftreffpunkt der Oberfläche des Werkstücks trifft.

Eine Strahlablenkeinheit kann vorteilhafter ein Galvanometerscanner sein. Ein Galvanometerscanner ist dem Fachmann als Vorrichtung bekannt, die den Laserstrahl mittels ein oder mehrerer Spiegel oder Prismen bewegt und die Bewegung der/des Spiegel(s) oder Prismas(en) mittels eines Galvanometerantriebes erzeugt.

Mit anderen Worten werden also die Laserlichtpulse sowohl in Richtung der Achse als auch in Rotationsrichtung bzw. in einer orthogonal zur Achse verlaufenden Richtung örtlich definiert auf die Oberfläche des Werkstücks appliziert. Der Laserstrahl entspricht bei der vorliegenden Erfindung keinem stetigen Photonenstrahl. Insofern stellt der Laserstrahl einen optischen Pfad dar, wobei entlang des optischen Pfades die Laserlichtpulse auf die Oberfläche des Werkstücks gegeben werden. Der Einfachheit halber wird im Rahmen der vorliegenden Erfindung auch von einem Laserstrahl gesprochen, wenn gerade keine Laserlichtpulse ausgesendet werden. Der Laserstrahl entspricht dann dem aktuellen optischen Pfad, der durch die momentane Bewegung bzw. Stellung der Strahlablenkeinheit definiert ist.

Ein Bewegen des Laserstrahls mittels der Strahlablenkeinheit auf der Oberfläche bedeutet insbesondere, dass der Laserstrahl bzw. die Laserlichtpulse so mittels der Strahlablenkeinheit abgelenkt wird, dass ein Auftreffpunkt des Laserstrahls (bzw. der einzelnen Laserlichtpulse) auf der Oberfläche des Werkstücks entlang der Achse bewegt wird.

Bei der Achse (die mit der Rotationsachse zusammenfällt) handelt es sich bevorzugt um eine Mittelachse bzw. Zylinderachse, wobei insbesondere die Oberfläche des Werkstücks koaxial bezüglich der Achse ausgebildet ist. Bei der Oberfläche des Werkstücks handelt es sich insbesondere (zumindest initial) um einen Zylindermantel.

Gemäß einer bevorzugten Ausführungsform handelt es sich bei dem Werkstück um einen rohrförmigen Rohling zur Herstellung eines Stents (z. B. für eine Herzklappenprothese oder in Form einer Gefäßstütze).

Unter einem Laserstrahlbearbeiten im Sinne der vorliegenden Erfindung werden alle Bearbeitungsvorgänge verstanden, bei denen durch die Laserlichtpulse eine Eigenschaft des Werkstücks, insbesondere dessen geometrische Form, verändert wird. Insbesondere wird bei der Laserstrahlbearbeitung Material des Werkstücks abgetragen. Das Laserstrahlbearbeiten kann daher auch ein Laserstrahlschneiden sein, bei dem z. B. Ausnehmungen, insbesondere Durchgangsöffnungen, in das Werkstück bzw. den Rohling geschnitten werden (durch Materialabtragung). Ein solches Laserstrahlbearbeiten bzw. Laserstrahlschneiden kann schichtweise erfolgen, d.h. eine Ausnehmung bzw. Durchgangsöffnung kann bei dem Verfahren schichtweise in das Werkstück bzw. in den Rohling eingebracht werden. Auf diese Weise kann z. B. eine Zellstruktur in den Rohling geschnitten werden, so dass letztlich ein Stent entsteht, der eine Vielzahl an integral miteinander verbundenen Streben aufweist. Die Streben stellen dabei den Teil des Werkstücks bzw. des Rohlings dar, der bei der Laserstrahlbearbeitung nicht bzw. nicht vollständig abgetragen worden ist.

Bei dem Antrieb kann es sich gemäß einer Ausführungsform des erfindungsgemäßen Verfahrens z. B. um einen Elektromotor handeln. Das Werkstück kann gemäß einer Ausführungsform des Verfahrens an einem Träger fixiert werden. Bei dem Träger kann es sich z. B. um einen zylinderförmigen Träger handeln, auf den das Werkstück anordenbar, insbesondere aufgeschoben werden kann. Der Träger kann insbesondere mit einer Welle des Antriebs bzw. Elektromotors gekoppelt und dadurch um die Achse rotierbar sein, so dass das am Träger fixierte Werkstück mittels des Antriebs um die Achse rotierbar ist. Die Welle kann ein Teil des Trägers sein bzw. den Träger bilden.

Die Strahlablenkeinheit ist gemäß einer Ausführungsform des erfindungsgemäßen Verfahrens zur Ablenkung des Laserstrahls in mindestens einer Dimension, insbesondere in mindestens zwei Dimensionen konfiguriert. Insbesondere weist die Strahlablenkeinheit zumindest einen beweglichen, vorzugsweise neigbaren oder rotierbaren Spiegel auf, der dazu konfiguriert ist, den Laserstrahl bzw. dessen Laserlichtpulse zu reflektieren um den Laserstrahl abzulenken. Um eine Ablenkung des Laserstrahls in zwei Dimensionen zu ermöglichen, kann die Strahlablenkeinheit zwei neigbare oder rotierbare Spiegel besitzen oder der Spiegel kann um zwei verschiedene Achsen neigbar bzw. rotierbar sein.

Alternativ hierzu kann die Strahlablenkeinheit zwei bewegliche, insbesondere neigbare oder rotierbare, Spiegel aufweisen, die jeweils um eine Achse neigbar sind, so dass der Laserstrahl wiederum in zwei Dimensionen ablenkbar ist. Die beiden Achsen können insbesondere orthogonal zueinander verlaufen.

Die Strahlablenkeinheit kann weiterhin auch einen rotierenden Polygonspiegel mit zumindest drei, insbesondere vier, insbesondere fünf oder sechs Facetten aufweisen. Insbesondere weist die Strahlablenkeinheit einen Polygonspiegel mit zumindest acht, zwölf oder 25 Facetten auf. Insbesondere kann der Polygonspiegel genau acht, zwölf oder 25 Facetten aufweisen.

In einigen Ausgestaltungen der Erfindung können auch ein Prisma oder mehrere Prismen anstelle des/der Spiegel(s) sinnvoll sein.

Weiterhin weist die Strahlablenkeinheit gemäß einer Ausführungsform des erfindungsgemäßen Verfahrens zum Bewegen bzw. Neigen des jeweiligen Spiegels bzw. zum Rotieren des Polygonspiegels einen Aktuator auf.

Weiterhin ist gemäß einer Ausführungsform des erfindungsgemäßen Verfahrens vorgesehen, dass zur Synchronisation der Lasereinrichtung mit der Strahlablenkeinheit eine Bewegung der Strahlablenkeinheit, die die Bewegung des Laserstrahls auf der Oberfläche des Werkstücks entlang der Achse erzeugt, mit den Laserlichtpulsen synchronisiert wird, wobei ein Signal zur Steuerung der Strahlablenkeinheit an die Laserlichtpulse oder an ein durch die Lasereinrichtung erzeugtes Laserlichtpulssignal gebunden wird. Das bedeutet insbesondere, dass eine konstante (sowie insbesondere einstellbare) Phasendifferenz bzw. Phasenbeziehung zwischen dem Signal zur Steuerung der Strahlablenkeinheit und den Laserlichtpulsen oder dem Laserlichtpulssignal vorliegt.

Weiterhin weist die Strahlablenkeinheit gemäß einer Ausführungsform des erfindungsgemäßen Verfahrens eine Steuereinheit auf, die dazu konfiguriert ist die Strahlablenkeinheit bzw. eine Bewegung der Strahlablenkeinheit bzw. des mindestens eines Spiegels der Strahlablenkeinheit zu steuern.

Die Strahlablenkeinheit weist vorzugsweise eine getaktete Steuereinheit auf. Insbesondere wird hierbei die Ausführung von Befehlen und/oder einer Bewegung der Strahlablenkeinheit in der Steuereinheit mit einem Takt der Steuereinheit synchronisiert. Dies ermöglicht eine zeitgesteuerte Ablenkung des Laserstrahls bzw. eine zeitgesteuerte Bewegung der Strahlablenkeinheit bzw. mindestens eines Spiegels der Strahlablenkeinheit. Der Takt der Laserscannersteuereinheit wird vorzugsweise mittels des besagten Laserscannersteuersignals gesteuert.

Weiterhin ist gemäß einer Ausführungsform des erfindungsgemäßen Verfahrens vorgesehen, dass zur Synchronisation der Lasereinrichtung mit dem Antrieb ein Antriebsteuersignal zur Steuerung des Antriebs bzw. der besagten Rotation des Werkstücks um die Achse an die Laserlichtpulse oder an das Laserlichtpulssignal gebunden wird. Das bedeutet insbesondere wiederum, dass eine konstante (sowie insbesondere einstellbare) Phasendifferenz bzw. Phasenbeziehung zwischen dem Antriebsteuersignal und den Laserlichtpulsen oder dem Laserlichtpulssignal vorliegt.

Zur Steuerung der Rotation des Werkstücks weist der Antrieb gemäß einer Ausführungsform des erfindungsgemäßen Verfahrens eine Antriebsteuereinheit auf, insbesondere in Form einer getakteten Antriebsteuereinheit. Insbesondere wird hierbei die Ausführung von Befehlen und/oder einer Bewegung des Antriebs zur Erzeugung der Rotation des Werkstücks mit einem Takt der Antriebsteuereinheit synchronisiert. Dies ermöglicht eine zeitgesteuerte Rotation des Antriebs. Der Takt der Antriebssteuereinheit wird vorzugsweise durch das besagte Antriebsteuersignal gesteuert.

Weiterhin ist gemäß einer Ausführungsform des erfindungsgemäßen Verfahrens vorgesehen, dass eine Phase des Steuersignals der Strahlablenkeinheit und/oder eine Phase des Antriebsteuersignals so eingestellt wird, dass der jeweilige Laserlichtpuls die Oberfläche des Werkstücks in dem gewünschten Auftreffpunkt trifft.

Zur Einstellung der Phase des Steuersignals der Strahlablenkeinheit und/oder des Antriebsteuersignals bezüglich der Phase der Laserlichtpulse bzw. des Laserlichtpulssignals kann insbesondere eine Abweichung zwischen der gewünschten Auftreffposition und einer tatsächlichen Auftreffposition des jeweiligen Laserlichtpulses bzw. des Laserstrahls auf der Oberfläche des zu bearbeitenden Werkstücks erfasst werden. Insbesondere steht die Abweichung für einen Abstand zwischen der gewünschten Auftreffposition und der tatsächlichen Auftreffposition. Dabei wird die Abweichung bzw. Entfernung in Bezug auf das Zentrum eines auf der Oberfläche erzeugten Laserspots und der gewünschten Auftreffposition gemessen.

Vorzugsweise wird die Phase des Steuersignals der Strahlablenkeinheit und/oder des Antriebsteuersignals in einem iterativen Prozess so lange eingestellt, bis die Abweichung zwischen der gewünschten Auftreffposition (Sollwert) und der tatsächlichen Auftreffposition (Istwert) einen vordefinierten Schwellwert unterschreitet. Der Schwellwert hängt insbesondere von der gewünschten Präzision der Einrichtung zur Laserstrahlbearbeitung ab. Gemäß einer bevorzugten Ausführungsform des erfindungsgemäßen Verfahrens ist der Schwellwert kleiner oder gleich dem einfachen Durchmesser des auf der Oberfläche durch den jeweiligen Laserlichtpuls erzeugten Laserspots, insbesondere kleiner oder gleich dem 0,5-fachen Durchmesser, insbesondere kleiner oder gleich dem 0,1-fachen Durchmesser, insbesondere kleiner oder gleich dem 0,01-fachen Durchmesser.

Die Einrichtung zur Laserstrahlbearbeitung weist gemäß einer Ausführungsform des erfindungsgemäßen Verfahrens zur Synchronisation der Lasereinrichtung mit der Strahlablenkeinheit und dem Antrieb eine Steuereinheit auf, die gemäß einer Ausführungsform des erfindungsgemäßen Verfahrens einen Phasenregelkreis (phase locked loop oder kurz PLL) aufweist. Insbesondere ist der Phasenregelkreis dazu ausgebildet, ein periodisches Ausgangssignal zu erzeugen, dessen Phase auf die Phase eines periodischen Eingangssignals bezogen ist. Der Phasenregelkreis kann einen Phasendetektor und einen Oszillator mit variabler Frequenz aufweisen, der ein periodisches Oszillatorsignal erzeugen kann. Der Phasendetektor vergleicht die Phase des periodischen Oszillatorsignals mit der Phase des Eingangssignals und passt den Oszillator so an, dass die beiden Phasen in einer vordefinierten Beziehung zueinander stehen.

Das periodische Oszillatorsignal oder ein davon abgeleitetes Oszillatorsignal kann somit als ein periodisches Ausgangssignal verwendet werden, das einen festen Phasenbezug zum periodischen Eingangssignal hat. Insbesondere liegt die Abtastrate des Phasendetektors bei 1 MHz bis 250 MHz, insbesondere bei 40 MHz bis 160 MHz. Folglich ist in diesem Fall die Frequenz des periodischen Ausgangssignals gleich der Frequenz des periodischen Eingangssignals. Allerdings kann eine Phase des periodischen Ausgangssignals gegenüber dem periodischen Eingangssignal um einen gewünschten Phasenwinkel verschoben werden.

Insbesondere wird das Laserlichtpulssignal als Eingangssignal des Phasenregelkreises verwendet. Das Laserlichtpulssignal kann z. B. direkt von der Lasereinrichtung oder von einem Triggersignal der Lasereinrichtung abgeleitet werden.

Gemäß einer weiteren Ausführungsform der Erfindung kann das periodische Ausgangssignal als besagtes Steuersignal der Strahlablenkeinheit verwendet werden, z. B. zur Steuerung eines Taktes der getakteten Steuereinheit der Strahlablenkeinheit. Werden Bewegungen der Strahlablenkeinheit durch die getaktete Steuereinheit der Strahlablenkeinheit gesteuert, wird eine Ablenkung des Laserstrahls oder eine Bewegung der Strahlablenkeinheit bzw. mindestens eines Spiegels der Strahlablenkeinheit mit den Laserlichtpulsen bzw. dem Laserlichtpulssignal synchronisiert.

Falls vorhanden, kann ein optischer Schalter der Lasereinrichtung in analoger Weise synchronisiert werden. Es kann jedoch ein unterschiedliches Phasenverhältnis zwischen der Phase der Laserlichtpulse bzw. dem Laserlichtpulssignal und dem optischen Schalter vorliegen.

Insbesondere verfügt die Steuereinheit des Laserbearbeitungsgerätes über einen Speicher zur Speicherung einer Befehlsliste bzw. zum Speichern von zum Bearbeiten des Werkstücks benötigten Daten. Durch die Ausführung der Befehle können Bewegungen der Strahlablenkeinheit, die Betätigung des optischen Schalters und/oder eine Rotation des Werkstücks um die Achse gesteuert werden. Wird das Laserbearbeitungsgerät z. B. zum Schneiden eines Stents eingesetzt, kann die Befehlsliste z. B. von einer Software erstellt werden, die in der Lage ist, eine entsprechende Vorlage in entsprechende Anweisungen für die Strahlablenkeinheit zu konvertieren.

Weiterhin ist gemäß einer Ausführungsform der Erfindung ebenfalls vorgesehen, dass die Steuereinheit bzw. der PLL ein weiteres periodisches Ausgangssignal bereitstellt, das ebenfalls einen festen Phasenbezug zum periodischen Eingangssignal (Laserlichtpulse bzw. Laserlichtpulssignal) aufweist. Das weitere Ausgangssignal kann von dem Ausgangssignal für die Strahlablenkeinheit abgeleitet sein. Auch hier kann eine Phase des weiteren periodischen Ausgangssignals gegenüber dem periodischen Eingangssignal um einen gewünschten Phasenwinkel verschoben werden.

Gemäß einer weiteren Ausführungsform des erfindungsgemäßen Verfahrens ist vorgesehen, dass das weitere periodische Ausgangssignal des Phasenregelkreises der Steuereinheit als besagtes Antriebsteuersignal zum Steuern des Antriebs verwendet wird, z. B. zur Steuerung eines Taktes der getakteten Steuereinheit des Antriebs. Eine Rotation des Werkstücks kann somit ebenfalls mit den Laserlichtpulsen und damit auch mit der Strahlablenkeinheit synchronisiert werden. Gibt die Antriebsteuereinheinheit z. B. ein gepulstes bzw. periodisches Signal an den Antrieb weiter, wobei die einzelnen Pulse oder Perioden des Signals die Welle des Antriebs, insbesondere Elektromotors, um einen definierten Winkel weiterdrehen, so wird eine Rotation des Werkstücks um die besagte Achse mit den Laserlichtpulsen bzw. dem Laserlichtpulssignal synchronisiert.

Gemäß einer weiteren Ausführungsform des erfindungsgemäßen Verfahrens ist vorgesehen, dass das Werkstück beim Bearbeiten des Werkstücks mit den Laserlichtpulsen (bzw. beim Bewegen des Laserstrahls auf der Oberfläche des Werkstücks entlang der Achse des Werkstücks) mit einer konstanten Rotationsgeschwindigkeit um die Achse (mittels des Antriebs) rotiert wird.

Gemäß einer Ausführungsform der Erfindung liegt die Rotationsgeschwindigkeit in einem Bereich von 0.1 Umdrehungen pro Sekunde bis 10 Umdrehungen pro Sekunde , insbesondere 0.3 Umdrehungen pro Sekunde bis 3 Umdrehungen pro Sekunde.

Die Lasereinrichtung ist insbesondere dazu konfiguriert, Laserlichtpulse mit einer Pulsdauer und einer Wiederholrate zu emittieren. Dabei liegt also die optische Leistung in Laserlichtpulsen von vordefinierter Dauer und mit vorgegebener Wiederholrate vor.

Gemäß einer weiteren Ausführungsform des erfindungsgemäßen Verfahrens ist vorgesehen, dass die Laserlichtpulse ultrakurze Laserlichtpulse sind, d. h. die Lasereinrichtung ist dazu konfiguriert, einen Laserstrahl von ultrakurzen Laserlichtpulsen zum Laserstrahlbearbeiten des Werkstücks zu erzeugen.

Gemäß einer Ausführungsform des erfindungsgemäßen Verfahrens ist vorgesehen, dass die Pulsdauer der von der Lasereinrichtung ausgesendeten Laserlichtpulse im Bereich von 1 fs bis 900 ps, vorzugsweise im Bereich von 100 fs bis 100 ps, insbesondere im Bereich von 200 fs bis 50 ps liegt. Laserlichtpulse mit den vorgenannten Pulslängen werden üblicherweise als ultrakurze Laserlichtpulse bezeichnet.

Darüber hinaus liegt die Wellenlänge der von der Lasereinrichtung emittierten Laserlichtpulse gemäß einer Ausführungsform im Bereich von 100 nm bis 4000 nm, insbesondere im Bereich von 300 nm bis 1100 nm. Die Wellenlänge beträgt z. B. 343 nm, 355 nm, 515 nm, 532 nm, 1030 nm oder 1064 nm.

Eine Spotgröße (Durchmesser) des Laserstrahls bzw. der einzelnen Laserlichtpulse liegt gemäß einer Ausführungsform der Erfindung auf der Oberfläche des Werkstücks in einem Bereich von etwa 1 µm bis 1000 µm, insbesondere 3 µm bis 100 µm. Zum Beispiel beträgt eine typische Spotgröße bei einer Wellenlänge von 532 nm 10 µm.

Weiterhin ist gemäß einer Ausführungsform des erfindungsgemäßen Verfahrens vorgesehen, dass die Lasereinrichtung einen Master-Oszillator aufweist. Insbesondere weist die Lasereinrichtung einen modengekoppelten Seed-Laser, insbesondere einen passiven modengekoppelten Seed-Laser, auf, gefolgt von einem Verstärker, wobei der Verstärker den Laserstrahl bzw. die Laserlichtpulse zur Bearbeitung des Werkstücks emittiert. Das heißt, die Lasereinrichtung basiert in diesem Fall auf einer Master-Oszillator-Leistungsverstärker-Anordnung (MOPA). Solche Lasereinrichtungen haben sich in der Laserbearbeitung als besonders nützlich erwiesen.

Vorzugsweise stellt eine Frequenz des Master-Oszillators einen integralen Faktor der Frequenz der vom Verstärker ausgesendeten Laserlichtpulse dar. Insbesondere liegt eine Frequenz (Wiederholrate) der Laserlichtpulse im Bereich von 1 Hz bis 1000 MHz, insbesondere 50 Hz bis 100 MHz, vorzugsweise 50 kHz bis 20 MHz. Eine Frequenz des Master-Oszillators (Seed-Laser) liegt vorzugsweise im Bereich von 10 MHz bis 1000 MHz, insbesondere 25 MHz bis 160 MHz. Die Frequenz des Master-Oszillators beträgt z. B. 82 MHz.

Weiterhin ist gemäß einer Ausführungsform des erfindungsgemäßen Verfahrens vorgesehen, dass einzelne oder mehrere aufeinanderfolgende Laserlichtpulse von der Einrichtung zur Laserstrahlbearbeitung bzw. der Lasereinrichtung blockiert werden, und/oder dass ein Laserlichtpuls oder mehrere Laserlichtpulse von der Einrichtung zur Laserstrahlbearbeitung bzw. der Lasereinrichtung durch Blockieren zumindest eines vorangehenden und zumindest eines nachfolgenden Laserlichtpulses einzeln bzw. als Gruppe ausgegeben werden.

Die Einrichtung zur Laserstrahlbearbeitung, insbesondere die Lasereinrichtung, kann hierfür einen optischen Schalter aufweisen (sogenannter PoD (Pulse on Demand), der im Strahlengang hinter der Lasereinrichtung angeordnet ist und so konfiguriert ist, dass er einzelne Laserlichtpulse des Laserstrahls zur Bearbeitung des Werkstücks definiert blockiert oder passieren lässt, wobei der optische Schalter insbesondere in Bezug auf die Laserlichtpulse oder das Laserlichtpulssignal synchronisiert ist). Der optische Schalter kann z. B. einen akusto-optischen Modulator (AOM) und/oder einen elektro-optischen Modulator (EOM) aufweisen. Mit einem solchen System können die Laserlichtpulse kontrolliert ein- und ausgeschaltet werden.

Weiterhin ist gemäß einer Ausführungsform des erfindungsgemäßen Verfahrens vorgesehen, dass der Laserstrahl entlang der Achse auf der Oberfläche des rotierenden Werkstücks vor und zurück bewegt wird (und zwar insbesondere über eine gesamte Länge des Werkstücks entlang der Achse des Werkstücks), wobei
- lediglich bei der jeweiligen Vorbewegung Laserlichtpulse zum Bearbeiten der Oberfläche des Werkstücks auf die Oberfläche des Werkstücks gegeben werden (und insbesondere dort im jeweiligen Auftreffpunkt auf der Oberfläche des Werkstücks auftreffen), oder
- lediglich bei der jeweiligen Rückbewegung Laserlichtpulse zum Bearbeiten der Oberfläche des Werkstücks auf die Oberfläche des Werkstücks gegeben werden (und insbesondere dort im jeweiligen Auftreffpunkt auf der Oberfläche des Werkstücks auftreffen), oder
- bei der jeweiligen Vorbewegung und/oder der jeweiligen Rückbewegung Laserlichtpulse zum Bearbeiten der Oberfläche des Werkstücks auf die Oberfläche gegeben werden (und dort insbesondere im jeweiligen Auftreffpunkt auf der Oberfläche des Werkstücks auftreffen).

Durch das Beschicken der Oberfläche des Werkstücks mit den Laserlichtpulsen während der Laserstrahl entlang der Achse des Werkstücks bei rotierendem Werkstück über die Oberfläche bewegt wird, entspricht der Verlauf einer Spur der Laserlichtpulse bzw. des Laserstrahls auf der Oberfläche des Werkstücks bei einer eindimensionalen, linearen Bewegung des Laserstrahls bzw. der Auftreffpunkte der Laserlichtpulse auf der Oberfläche des Werkstücks keiner Geraden, die parallel zur Achse verläuft, sondern (bei zylindrischer und zur Achse koaxialer Oberfläche) einem Schraubenlinienabschnitt, der in Näherung einer zur Achse windschiefen Geraden entspricht.

Derartige Abweichungen können durch eine entsprechende Bewegung des Laserstrahls bzw. der Auftreffpunkte in einer zur Achse orthogonal erstreckten Richtung kompensiert werden.

Gemäß einer weiteren Ausführungsform des erfindungsgemäßen Verfahrens ist daher vorgesehen, dass der Laserstrahl mittels der Strahlablenkeinheit zusätzlich auf der Oberfläche des Werkstücks entlang der orthogonal zur Achse erstreckten Richtung bewegt wird (d. h. der jeweilige Auftreffpunkt wird in Umfangsrichtung bzw. entlang der Rotationsrichtung des Werkstücks auf der rotierenden Oberfläche des Werkstücks bewegt, z. B. in Rotationsrichtung oder entgegen der Rotationsrichtung), und zwar insbesondere zum Kompensieren der Rotation des Werkstücks oder zur Erzeugung eines Hinterschnitts. Letzteres ist möglich, da der Laserstrahl durch eine Bewegung in der besagten orthogonalen Richtung nicht mehr normal auf die (zylindrische) Oberfläche auftrifft, sondern unter einem spitzen Winkel.

Weiterhin ist gemäß einer Ausführungsform des erfindungsgemäßen Verfahrens vorgesehen, dass das Material des Werkstücks bei um die Achse kontinuierlich rotierendem Werkstück, insbesondere unter Hin- und Herbewegen des Laserstrahls auf der Oberfläche entlang der Achse, schichtweise nach und nach mittels der Laserlichtpulse abgetragen wird.

Weiterhin ist gemäß einer Ausführungsform des erfindungsgemäßen Verfahrens vorgesehen, dass Material des Werkstücks mittels der Laserlichtpulse derart abgetragen wird, dass eine Wand des Werkstücks (insbesondere in Form eines zylindrischen Rohlings für einen Stent) unter Ausbildung zumindest einer Durchgangsöffnung oder einer Vielzahl an Durchgangsöffnungen durchbrochen wird. Hierdurch kann eine vordefinierte Stentgeometrie erzeugt werden, indem nämlich eine Mehrzahl an entsprechend geformten Durchgangsöffnungen, die später die Zellen des Stents bilden, in das Werkstück eingebracht bzw. geschnitten werden.

Das erfindungsgemäße Verfahren eröffnet mit Vorteil neue Möglichkeiten im Design für Stents, da Schnitte, die einer Kontur folgen, prozessbedingt nicht mehr erforderlich sind, sondern beispielsweise auch Schrägen erzeugt werden können, und Material nur teilweise entfernt oder eine Oberflächenstrukturierung aufgebracht werden kann.

Weiterhin ist gemäß einer Ausführungsform des erfindungsgemäßen Verfahrens vorgesehen, dass geometrische Daten (z. B. entsprechend einem Schnittmuster) für die Laserbearbeitung des Werkstücks als Schwarz-Weiß-Bitmap, Graustufen-Bitmap, oder als Vektor-Graphik bereitgestellt (bzw. an die Einrichtung zur Laserbearbeitung übertragen) werden.
Schwarz und Weiß repräsentieren dabei Laserlichtpuls an (durchgelassener Laserlichtpuls) bzw. aus (blockierter Laserlichtpuls). Die Übertragung der geometrischen Daten des zu bearbeitenden Werkstücks an die Einrichtung zur Laserbearbeitung kann aber grundsätzlich auch in anderen Formaten erfolgen.

Weiterhin ist gemäß einer Ausführungsform des erfindungsgemäßen Verfahrens vorgesehen, dass der Laserstrahl entlang der Achse auf der Oberfläche des Werkstücks mit einer Geschwindigkeit im Bereich von 10 m/s bis 40 m/s bewegt wird, insbesondere im Bereich von 20 m/s bis 40 m/s, insbesondere im Bereich von 20 m/s bis 30 m/s. Die Geschwindigkeit beträgt z. B. 25 m/s. Insbesondere wird beispielsweise der Laserstrahl bei einer Brennweite der Lasereinrichtung von 100 mm mit einer Geschwindigkeit von bis zu 25 m/s auf der Oberfläche entlang der Achse bewegt oder bei einer Brennweite von 160 mm mit einer Geschwindigkeit von bis zu 40 m/s.

Die Geschwindigkeit des Laserstrahls meint hierbei die Geschwindigkeit mit der der Auftreffpunkt des Laserstrahls bzw. der einzelnen Laserlichtpulse auf der Oberfläche des Werkstücks (durch eine entsprechende Bewegung der Strahlablenkeinheit) entlang der Achse bewegt wird.

Weiterhin ist gemäß einer Ausführungsform des erfindungsgemäßen Verfahrens vorgesehen, dass das Werkstück ein Rohling für einen Stent ist, wobei insbesondere durch die Laserstrahlbearbeitung aus dem Werkstück ein Stent hergestellt wird.

Gemäß einem weiteren Aspekt betrifft die vorliegende Erfindung eine Einrichtung zur Laserstrahlbearbeitung, die bevorzugt bei der Durchführung des erfindungsgemäßen Verfahrens verwendet wird. Diese Einrichtung weist zumindest auf:
- eine Lasereinrichtung, die dazu konfiguriert ist, einen Laserstrahl in Form von aufeinanderfolgenden Laserlichtpulsen zum Laserstrahlbearbeiten des Werkstücks zu erzeugen,
- einem Träger zum Halten des Werkstücks, wobei das Werkstück am Träger fixierbar ist,
- einen mit dem Träger gekoppelten Antrieb, der zum kontinuierlichen Rotieren des Werkstücks um eine Achse des Werkstücks in einer Rotationsrichtung mit einer (z. B. konstanten) Rotationsgeschwindigkeit ausgebildet ist, und
- einer Strahlablenkeinheit, die dazu konfiguriert ist, den Laserstrahl auf eine Oberfläche des Werkstücks abzulenken und auf der Oberfläche entlang der Achse des Werkstücks zu bewegen,
wobei die Einrichtung eine Steuereinheit aufweist, die dazu konfiguriert ist, die Lasereinrichtung mit der Strahlablenkeinheit und mit dem Antrieb so zu synchronisieren, dass der jeweilige Laserlichtpuls die Oberfläche des Werkstücks in einem gewünschten (d. h. vordefinierten) Auftreffpunkt der Oberfläche des Werkstücks trifft, wenn das Werkstück am Träger fixiert ist und mit dem Antrieb um die Achse (bei insbesondere konstanter Rotationsgeschwindigkeit) kontinuierlich rotiert wird.

Im Sinne der vorliegenden Erfindung ist jegliche Fixierung des Werkstücks am Träger als ausreichend anzusehen, die ein Rotieren des Werkstücks um die Achse gestattet, so dass sich das Werkstück im Wesentlichen nicht bezüglich des Trägers verschiebt oder in sonstiger Weise verlagert. Das Werkstück kann z. B. durch eine Presspassung auf dem Träger gehalten werden. Andere Fixierungen sind ebenfalls denkbar.

Die oben im Zusammenhang mit dem Verfahren beschriebenen Merkmale der Einrichtung zur Laserstrahlbearbeitung bzw. der einzelnen Komponenten der Einrichtung stellen ebenfalls Ausführungsformen der erfindungsgemäßen Einrichtung dar und können insofern zur näheren Charakterisierung der Einrichtung dienen.

Da bei dem erfindungsgemäßen Verfahren die Laserstrahlbearbeitung insbesondere schichtweise erfolgt, können grundsätzliche alle erdenklichen Geometrien bearbeitet werden. Darüber hinaus ist jede Bearbeitung von Rundmaterial möglich, vom Strukturieren von Oberflächen bis zum Bohren von Rundfiltern.

Zurzeit werden Gefässstützen/Stents bereits mit Laserstrahlung durch Schneiden hergestellt. Dabei wird der Stent über eine Linearachse in axialer Richtung und über eine Drehachse in azimutaler Richtung unter dem stehenden Laserstrahl bewegt. Die Geschwindigkeit des Bearbeitungsprozesses, und damit die Bearbeitungsdauer, ist von der Dynamik der Achsen abhängig. Mit dem Schneiden durch Abtragen während des Scannens, wie es insbesondere in der vorliegenden Erfindung vorgeschlagen wird, wird diese Begrenzung aufgehoben, die Prozessgeschwindigkeit kann prinzipiell mit der verfügbaren mittleren Laserleistung skaliert werden.

Die vorliegend beschriebene Erfindung stellt damit eine Möglichkeit dar, Stents mit hoher Genauigkeit und Flexibilität zu fertigen. Entscheidend ist hierbei die Synchronisation der Lasereinrichtung mit der Strahlablenkeinheit einerseits und dem Antrieb für die Rotation des Werkstücks andererseits. Ohne diese Synchronisation ist eine ausreichende Genauigkeit nicht erreichbar. Zusätzlich bietet diese Synchronisation die Möglichkeit, höhere Repetitionsraten, und damit auch höhere mittlere Laserleistungen, zu verwenden. Die Nutzung des Bitmap-Modus konventioneller Steuerungen von Strahlablenkeinheiten ist für Ultrakurzpulslaser nur begrenzt möglich, da eine Interpretation der Graustufen erfolgen muss. Prinzipiell kann eine bestimmte Graustufe z. B. über eine angepasste Pulslänge, über die Pulsenergie oder über die Anzahl der Laserlichtpulse pro Fläche eingestellt werden. Um die erfindungsgemäße Einrichtung bzw. das erfindungsgenmäße Verfahren effizient nutzen zu können, wird mit Vorteil der Laserstrahl mit konstanter Geschwindigkeit über die Oberfläche des Werkstücks bewegt. Dies ist insbesondere möglich, da die Lasereinrichtung (siehe auch oben) für jedes einzelne Pixel an- oder ausgeschaltet werden kann, ohne dass sich die Geschwindigkeit der Strahlbewegung ändert.

Aufgrund der erfindungsgemäßen Synchronisation erlaubt die Erfindung weiterhin einen nahtlosen Übergang von einer Umdrehung des Werkstücks zur nächsten, so dass bei einem kontinuierlich rotierenden Werkstück, das in Schichten bearbeitet wird, eine ausreichend hohe Absolutgenauigkeit erreichbar ist.

Weitere Merkmale und Ausführungsformen der vorliegenden Erfindung sollen nachfolgend im Zusammenhang mit den Figuren beschrieben werden. Es zeigen:
- Fig. 1: eine schematische Darstellung des erfindungsgemäßen Verfahrens bzw. einer erfindungsgemäßen Einrichtung zur Laserstrahlbearbeitung eines Werkstücks, insbesondere in Form eines zylindrischen (z. B. metallischen) Rohlings für einen Stent; und
- Fig. 2: eine grafische Darstellung einer mit dem Verfahren erzielbaren Bearbeitungsdauer beim Stentschneiden in Abhängigkeit der mittleren Leistung der Lasereinrichtung.

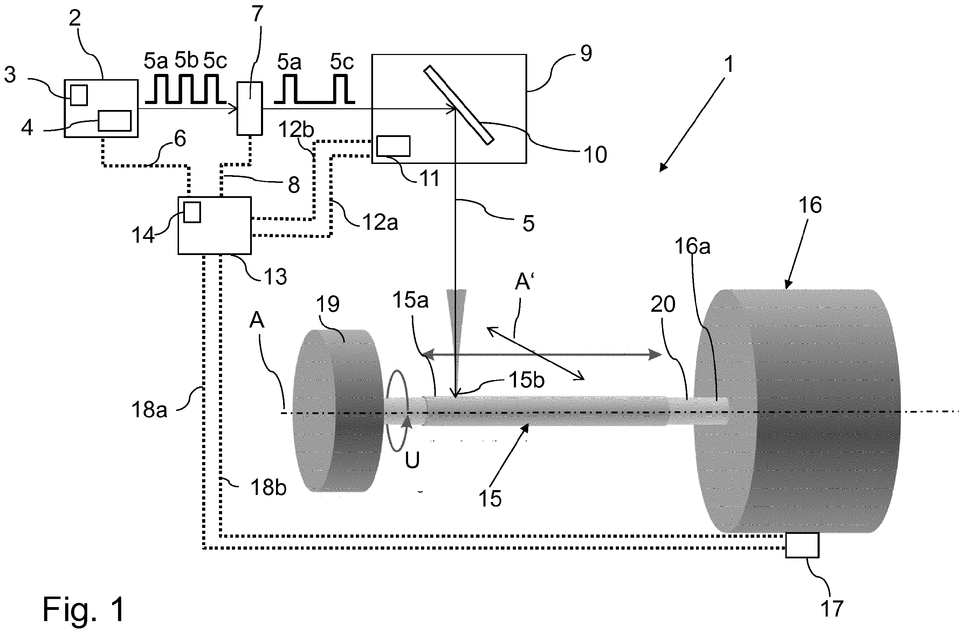

Fig. 1 zeigt eine Ausführungsform einer erfindungsgemäßen Einrichtung 1 zur Laserstrahlbearbeitung. Die Einrichtung 1 weist eine Lasereinrichtung 2 zur Erzeugung eines ablenkbaren Laserstrahls 5 aus aufeinander folgenden Laserlichtpulsen 5a, 5b, 5c auf.

Die Lasereinrichtung 2 kann auf einer MOPA-Anordnung (Master Oscillator Power Amplifier) basieren und umfasst dann einen modengekoppelten Seed-Laser (Master-Oszillator) 3, der z. B. mit einer Frequenz von 82 MHz betrieben wird, sowie einen Leistungsverstärker (Power Amplifier) 4, der z. B. mit einer Frequenz von 500 kHz betrieben wird. Die Lasereinrichtung 2 kann z. B. einen Laserstrahl 5 von Laserlichtpulsen 5a, 5b, 5c in Form von ultrakurzem Laserlichtpulsen 5a, 5b, 5c mit einer Wellenlänge von 532 nm und einer Pulsdauer von ca. 10 ps emittieren.

Eine Energie pro Laserlichtpuls 5a, 5b, 5c kann dabei z. B. von normalerweise einigen µJ bis zu maximal 200 mJ betragen. In Fig. 1 sind als Beispiel drei aufeinanderfolgende Laserlichtpulse 5a, 5b, 5c dargestellt. Die Lasereinrichtung 2 generiert des Weiteren ein Laserlichtpulssignal (Seed-Frequenz) 6. Dabei entspricht bzw. erzeugt jeder Laserlichtpuls 5a, 5b, 5c einen zugeordneten Puls dieses Laserlichtpulssignals 6.

Wie weiterhin in Fig. 1 gezeigt ist, kann die Einrichtung 1 einen optischen Schalter (PoD) 7 aufweisen, der im Strahlengang der Lasereinrichtung 2 vor einer Strahlablenkeinheit 9 angeordnet ist (siehe unten), so dass der Laserstrahl 5 den Schalter 7 passiert. Der Schalter 7 kann als ein akusto-optischer Modulator (AOM) ausgestaltet sein, der auf Basis eines Schaltsignals 8 in der Lage ist, den Laserstrahl 5 bzw. einzelne Laserlichtpulse 5a, 5b, 5c zu blockieren oder passieren zu lassen. So können mit dem optischen Schalter 7 z. B. einzelne Pulse 5a, 5c aus dem Laserstrahl 5 der Laserpulse 5a, 5b, 5c ausgewählt werden. Der optische Schalter 7 kann bereits in der Lasereinrichtung integriert sein.

Nach Passieren des optischen Schalters 7 wird der Strahl 5 der Laserlichtpulse 5a, 5b, 5c bzw. der selektierten Pulse 5a, 5c von einer Strahlablenkeinheit 9 abgelenkt. Die Strahlablenkeinheit 9 kann z. B. einen in zwei Dimensionen neigbaren Spiegel 10 aufweisen, so dass der Laserstrahl 5 bzw. der Auftreffpunkt 15b des jeweiligen Laserlichtpulses 5a, 5c entlang einer Achse A des zu bearbeitenden Werkstücks 15 auf der Oberfläche 15a des Werkstücks 15 bewegbar ist sowie entlang einer zur Achse A orthogonalen Richtung A'. Die Strahlablenkeinheit 9 ist hierzu mittels einer Steuereinheit (Controller-Karte, z. B. RTC5 oder RTC6) 11 steuerbar.

Der Laserstrahl 5 kann vor der Strahlablenkeinheit 9 durch zusätzliche refraktive oder diffraktive optische Elemente wie Linsen oder Spiegel angepasst werden und nach der Strahlablenkeinheit 9 eine weitere Optik passieren, die z. B. ein 100 mm telezentrisches F-Theta-Objektiv aufweisen kann (in der Fig. 1 nicht dargestellt) und trifft sodann auf die Oberfläche 15a des zu bearbeitenden, insbesondere metallischen Werkstücks 15, bei dem es sich insbesondere um einen zylindrischen Rohling für einen Stent handelt.

Das Wertstück 15 erstreckt sich entlang einer Achse A (hier eine Längs- bzw. Zylinderachse des Werkstücks 15), wobei die Einrichtung 1 einen Antrieb 16 aufweist, der zum Rotieren des Werkstücks 15 (und somit der koaxial zur Achse A angeordneten Oberfläche 15a) um die Achse A ausgebildet ist. Der Antrieb 16 kann hierzu eine Welle 16a aufweisen, die mit einem Träger 20 zum Halten des Werkstücks 15 verbunden ist. Weiterhin kann der Träger 20 bzw. die Welle 16a durch ein dem Antrieb 16 gegenüberliegendes Lager 19 gelagert sein.

Die Strahlablenkeinheit 9 und der Antrieb 16 der Einrichtung 1 werden mit einer Steuereinheit 13 gesteuert bzw. mit der Lasereinrichtung 2 synchronisiert. Hierdurch findet auch ein Synchronisieren der Strahlablenkeinheit 9 mit dem Antrieb 16 statt.

Die Steuereinheit 13 kann hierzu einen Phasenregelkreis (PLL) 14 aufweisen. Hierbei kann das Laserpulssignal 6 als Eingangssignal des PLL 14 verwendet werden. Das elektronische Schaltsignal 8 zur Ansteuerung des optischen Schalters 7 kann mit dem Laserpulssignal 6 synchronisiert bzw. phasenstarr an dieses gebunden werden. Der PLL 14 erzeugt außerdem ein Ausgangssignal 12a, mit dem ein Takt der getakteten Steuereinheit 11 der Strahlablenkeinheit gesteuert wird. Das Ausgangssignal 12a wird mittels des PLL 14 mit dem Laserlichtpulssignal 6 synchronisiert. Das Ausgangssignal 12a hat somit eine feste Phasenbeziehung zum Laserpulssignal 6 bzw. zu den Laserlichtpulsen 5a, 5b, 5c, .... Die Steuereinheit 13 ist jedoch so konfiguriert, dass eine Phasenverschiebung des Ausgangssignals 12a gegenüber dem Laserpulssignal 6 eingestellt werden kann. Zusätzlich kann die Steuereinheit 13 ein Startsignal 12b für die Strahlablenkeinheit 9 ausgeben. Das Startsignal 12b wird ebenfalls mit dem Ausgangssignal 12a bzw. dem Laserpulssignal 6 synchronisiert.

In ähnlicher Weise wird der Antrieb 16 mit der Lasereinrichtung 2 synchronisiert. Hierzu kann ein weiteres Ausgangssignal 18a mittels der Steuereinheit 13 bzw. des PLL 14 bereitgestellt werden, mit dem ein Takt einer getakteten Antriebsteuereinheit 17 des Antriebs 16 gesteuert wird. Das weitere Ausgangssignal 18a ist dabei ebenfalls mit dem Laserlichtpulssignal 6 synchronisiert und steht mit diesem (bzw. mit den Laserlichtpulsen) in einer festen Phasenbeziehung. Auch diesbezüglich ist die Steuereinheit 13 so konfiguriert, dass eine Phasenverschiebung des weiteren Ausgangssignals 18a gegenüber dem Laserlichtpulssignal 6 (unabhängig von dem Ausgangssignal 12a) eingestellt werden kann. Zusätzlich kann die Steuereinheit 13 ein weiteres Startsignal 18b für den Antrieb 16 ausgeben Das weitere Startsignal 18b wird ebenfalls mit dem weiteren Ausgangssignal 18a bzw. dem Laserpulssignal 6 synchronisiert. Das weitere Ausgangssignal 18a kann vom Ausgangssignal 12a abgeleitet sein, wobei die Steuereinheit 13 die Möglichkeit bietet, die Phase der beiden Signale 12a, 18a zum Kalibieren der Einrichtung 1 unabhängig voneinander einzustellen.

Durch die erfindungsgemäße Synchronisation der Lasereinrichtung 2 mit der Strahlablenkeinheit 9 und dem Antrieb 16 kann das kontinuierlich (mit insbesondere konstanter Geschwindigkeit) rotierende Werkstück 15 schichtweise bearbeitet werden, wobei der Laserstrahl 15 mittels der Strahlablenkeinheit 9 entlang der Achse A auf der Oberfläche 15a hin- und her bewegt wird (dargestellt durch den parallel zur Achse A eingetragenen Doppelpfeil in Fig. 1).

Zum Korrigieren eines Versatzes in Rotations- bzw. Umfangsrichtung U des Werkstücks 15 kann der Laserstrahl 5 insbesondere auch in einer zur Achse A orthogonalen Richtung A' verfahren werden (angezeigt durch einen entsprechenden Doppelpfeil in der Fig. 1), wobei diese Richtung A' insbesondere auch orthogonal zum Laserstrahl 5 verläuft, wenn dieser in einer Ebene liegt, die die Achse A aufweist.

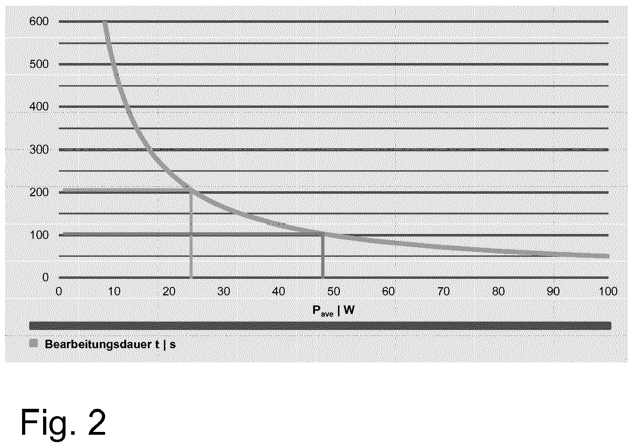

Die Bearbeitungsdauer der Laserstrahlbearbeitung des Werkstücks 15 bei dem erfindungsgemäßen Verfahren ist durch das gesamte abzutragende Volumen, d.h. die geometrischen Abmessungen des Stents und die Laserleistung definiert. Insbesondere hängt die Bearbeitungsdauer umgekehrt proportional von der mittleren Laserleistung Pₐᵥₑ ab, so dass eine Verdopplung der eingesetzten mittleren Laserleistung eine Halbierung der Bearbeitungsdauer bedeutet, wie insbesondere aus Figur 2 ersichtlich ist.

Die Geschwindigkeit, mit der der Laserstrahl 5 bewegt werden kann, ist z. B. durch den verwendeten Typ der Strahlablenkeinheit limitiert. Wird z. B. mit einem Galvanometerscanner gearbeitet, sind bei einer Brennweite von 100 mm zurzeit Geschwindigkeiten von bis zu 25 m/s, bei einer Brennweite von 160 mm von bis zu 40 m/s möglich.

Da die Strahlablenkeinheit 9 den Laserstrahl 5 in der Regel nur in geraden Linien bewegt (entlang der Achse A), reduzieren sich die Beschleunigungs- und Abbremsphasen. Das System kann so auf eine minimale Bearbeitungsdauer optimiert werden, so dass grundsätzlich für eine vorgegebene Scanlänge die optimale Geschwindigkeit gegeben ist.

**Tabelle 1:**

| | | | | | | |
|---|---|---|---|---|---|---|
| Stent Länge | 100 | mm | 20 | mm | 20 | mm |
| Stent Durchmesser | 3.5 | mm | 4.0 | mm | 2.0 | mm |
| Wandstärke | 0.165 | mm | 0.370 | mm | 0.186 | mm |
| Schnittlänge | 5638.16 | mm | 800.54 | mm | 707.80 | mm |
| aktuelle Schneiddauer | 842 | s | 242 | s | 206 | s |
| Schnittgeschwindigkeit | 6.7 | mm/s | 3.3 | mm/s | 3.4 | mm/s |
| | 401.8 | mm/min | 198.5 | mm/min | 206.2 | mm/min |
| spezifische Abtragrate | 0.18 | mm³/(min · W) | 0.18 | mm³/(min•W) | 0.26 | mm³/(min•W) |
| mittlere Laserleistung Pₐᵥₑ | 68 | W | 116 | W | 24 | W |

Tabelle 1 listet Daten typischer Stentgeometrien auf und zeigt, welche mittlere Laserleistung Pₐᵥₑ nötig ist, um die genannte Bearbeitungsdauer mit dem erfindungsgemäßen Verfahren zu erreichen. Derzeitige Ultrakurzpulslasersysteme können eine mittlere Laserleistung von über 100 W liefern, so dass die angegebenen Bearbeitungsdauern leicht erzielt werden können (vgl. hierzu auch Fig. 2). Da eine Verdopplung der mittleren Laserleistung eine Halbierung der Bearbeitungsdauer bedeutet, können mit verfügbaren Lasereinrichtungen 2 deutliche Steigerungen der Produktivität erreicht werden.

## Patentansprüche

1. Verfahren zum Laserstrahlbearbeiten eines entlang einer Achse (A) erstreckten Werkstücks (15) mittels einer Einrichtung zur Laserstrahlbearbeitung (1), wobei die Einrichtung zur Laserstrahlbearbeitung (1) eine Lasereinrichtung (2) aufweist, die dazu konfiguriert ist, einen Laserstrahl (5) von einzelnen aufeinanderfolgenden Laserlichtpulsen (5a, 5b, 5c) zu erzeugen, eine Strahlablenkeinheit (9), die dazu konfiguriert ist, den Laserstrahl (5) auf eine Oberfläche (15a) des Werkstücks (15) abzulenken und auf der Oberfläche (15a) entlang der Achse (A) zu bewegen, und einen Antrieb (16) zum kontinuierlichen Rotieren des Werkstücks (15) um die Achse (A) in einer Rotationsrichtung (U) mit einer Rotationsgeschwindigkeit, wobei zum Laserstrahlbearbeiten des Werkstücks (15) das Werkstück (15) mittels des Antriebs (16) in eine kontinuierliche Rotation in der Rotationsrichtung (U) um die Rotationsachse (A) versetzt wird und der Laserstrahl (5) mittels der Strahlablenkeinheit (9) auf die Oberfläche (15a) des Werkstücks (15) abgelenkt und auf der Oberfläche (15a) entlang der Achse (A) bewegt wird, wobei Material des Werkstücks (15) mittels Laserlichtpulsen (15a, 15c) abgetragen wird, und wobei die Lasereinrichtung (2) mit dem Laserscanner (9) und dem Antrieb (16) so synchronisiert ist, dass der jeweilige Laserlichtpuls (15a, 15c) die Oberfläche (15a) in einem gewünschten Auftreffpunkt (15b) der Oberfläche (15a) des Werkstücks (15) trifft.

2. Verfahren nach Anspruch 1, wobei zur Synchronisation der Lasereinrichtung (2) mit dem Laserscanner (9) eine Bewegung des Laserscanners (9), die die Bewegung des Laserstrahls (5) auf der Oberfläche (15a) des Werkstücks (15) entlang der Achse (A) erzeugt, mit den Laserlichtpulsen (5a, 5b, 5c) synchronisiert wird, wobei ein Steuersignal der Strahlablenkeinheit (12a) zur Steuerung der Strahlablenkeinheit (9) an die Laserlichtpulse (5a, 5b, 5c) oder an ein durch die Lasereinrichtung (2) erzeugtes Laserlichtpulssignal (6) gebunden wird.

3. Verfahren nach Anspruch 1 oder 2, wobei zur Synchronisation der Lasereinrichtung (2) mit dem Antrieb (16) ein Antriebsteuersignal (18a) zur Steuerung des Antriebs (16) an die Laserlichtpulse (5a, 5b, 5c) oder an ein durch die Lasereinrichtung (2) erzeugtes Laserlichtpulssignal (6) gebunden wird.

4. Verfahren nach Anspruch 2 oder 3, wobei eine Phase des Steuersignals der Strahlablenkeinheit (12a) und/oder eine Phase des Antriebsteuersignals (18a) so eingestellt wird, dass der jeweilige Laserlichtpuls (15a, 15c) die Oberfläche (15a) in dem gewünschten Auftreffpunkt (15b) trifft.

5. Verfahren nach einem der vorhergehenden Ansprüche, wobei das Werkstück (15) beim Bearbeiten des Werkstücks (15) mit den Laserlichtpulsen (15a, 15c) mit einer konstanten Rotationsgeschwindigkeit um die Achse (A) mittels des Antriebs (16) rotiert wird.

6. Verfahren nach einem der vorhergehenden Ansprüche, wobei die Laserlichtpulse (15a, 15b, 15c) ultrakurze Laserlichtpulse sind.

7. Verfahren nach einem der vorhergehenden Ansprüche, wobei zumindest ein Laserlichtpuls (15b) oder mehrere Laserlichtpulse von der Einrichtung zur Laserstrahlbearbeitung (1) blockiert werden, und/oder wobei ein Laserlichtpuls oder mehrere Laserlichtpulse von der Einrichtung zur Laserstrahlbearbeitung (1) jeweils durch Blockieren zumindest eines vorangehenden und zumindest eines nachfolgenden Laserlichtpulses einzeln oder in einer Gruppe ausgegeben werden.

8. Verfahren nach einem der vorhergehenden Ansprüche, wobei der Laserstrahl (5) entlang der Achse (A) auf der Oberfläche (15a) des rotierenden Werkstücks (15) mehrfach vor und zurück bewegt wird, wobei
- lediglich bei der jeweiligen Vorbewegung Laserlichtpulse zum Bearbeiten der Oberfläche (15a) des Werkstücks (15) auf die Oberfläche (15a) des Werkstücks (15) gegeben werden, oder
- lediglich bei der jeweiligen Rückbewegung Laserlichtpulse zum Bearbeiten der Oberfläche (15a) des Werkstücks (15) auf die Oberfläche (15a) des Werkstücks (15) gegeben werden, oder
- bei der jeweiligen Vorbewegung und/oder der jeweiligen Rückbewegung Laserlichtpulse zum Bearbeiten der Oberfläche (15a) des Werkstücks (15) auf die Oberfläche (15a) gegeben werden.

9. Verfahren nach einem der vorhergehenden Ansprüche, wobei der Laserstrahl (5) mittels der Strahlablenkeinheit (9) zusätzlich auf der Oberfläche (15a) des Werkstücks (15) entlang einer orthogonal zur Achse (A) erstreckten Richtung (A') bewegt wird.

10. Verfahren nach einem der vorhergehenden Ansprüche, wobei Material des Werkstücks (15) schichtweise nach und nach mittels der Laserlichtpulse (15a, 15c) abgetragen wird.

11. Verfahren nach einem der vorhergehenden Ansprüche, wobei Material des Werkstücks (15) mittels der Laserlichtpulse (15a, 15c) derart abgetragen wird, dass eine Wand des Werkstücks (15) unter Ausbildung zumindest einer Durchgangsöffnung oder einer Vielzahl an Durchgangsöffnungen durchbrochen wird.

12. Verfahren nach einem der vorhergehenden Ansprüche, wobei geometrische Daten für die Laserstrahlbearbeitung des Werkstücks als Schwarz-Weiß-Bitmap, Graustufen-Bitmap, oder als Vektor-Graphik bereitgestellt werden.

13. Verfahren nach einem der vorhergehenden Ansprüche, wobei der Laserstrahl (5) entlang der Achse (A) auf der Oberfläche (15a) des Werkstücks (15) mit einer Geschwindigkeit im Bereich von 10 m/s bis 40 m/s bewegt wird, insbesondere im Bereich von 20 m/s bis 40 m/s, insbesondere im Bereich von 20 m/s bis 30 m/s.

14. Verfahren nach einem der vorhergehenden Ansprüche, wobei das Werkstück (15) ein Rohling für einen Stent ist.

15. Einrichtung zur Laserstrahlbearbeitung (1), mit:
- einer Lasereinrichtung (2), die dazu konfiguriert ist, einen Laserstrahl (5) von Laserlichtpulsen (5a, 5b, 5c) zu erzeugen,
- einem Träger (20) zum Halten des Werkstücks (15), wobei der Träger (20) zum Fixieren des Werkstücks (15) am Träger (20) ausgebildet ist,
- einen mit dem Träger (20) gekoppelten Antrieb (16), der zum kontinuierlichen Rotieren des Werkstücks (15) um eine Achse (A) des Werkstücks (15) in einer Rotationsrichtung (U) mit einer Rotationsgeschwindigkeit ausgebildet ist, und
- einer Strahlablenkeinheit (9), die dazu konfiguriert ist, den Laserstrahl (5) auf eine Oberfläche (15a) des Werkstücks (15) abzulenken und auf der Oberfläche (15a) entlang der Achse (A) des Werkstücks zu bewegen, wenn das Werkstück (15) am Träger (20) fixiert ist,
wobei die Einrichtung (1) eine Steuereinheit (13) aufweist, die dazu konfiguriert ist, die Lasereinrichtung (2) mit der Strahlablenkeinheit (9) und mit dem Antrieb (16) so zu synchronisieren, dass der jeweilige Laserlichtpuls (15a, 15c) die Oberfläche (15a) des Werkstücks (15) in einem gewünschten Auftreffpunkt (15b) der Oberfläche (15a) des Werkstücks (15) trifft, wenn das Werkstück (15) am Träger (20) fixiert ist und mit dem Antrieb (16) um die Achse (A) rotiert wird.
